# EUROPEAN PATENT SPECIFICATION

(11) **EP 2 526 860 B1**
(45) Date of publication and mention of the grant of the patent: **15.10.2014**
(21) Application number: 10810730.1
(22) Date of filing: 04.02.2010
(51) Int. Cl.: A61B 5/0408, A61D 1/00

(54) **MULTIFUNCTIONAL CLAMP FOR VETERINARIANS**
MULTIFUNKTIONSKLAMMER FÜR TIERÄRZTE
CLAMP MULTIFONCTIONNEL POUR VÉTÉRINAIRES

(30) Priority: 22.01.2010 CN 201020103228 U
(43) Date of publication of application: 28.11.2012
(73) Proprietor: Edan Instruments, Inc., Shekou, Nanshan Shenzhen 518067 (CN)
(72) Inventor: WANG, Gonghua, Shenzhen Guangdong 518067 (CN); LIU, Gang, Shenzhen Guangdong 518067 (CN); XIE, Xicheng, Shenzhen Guangdong 518067 (CN)
(74) Representative: Jeannet, Olivier
(86) International application number: PCT/CN2010/070514
(87) International publication number: WO 2011/088625

(56) References cited:
- CN-U- 85 202 339
- CN-U- 85 202 644
- CN-Y- 2 845 719
- CN-Y- 201 167 950
- CN-Y- 201 312 804
- DE-C1- 19 643 988
- US-A- 4 640 563
- US-A1- 2009 062 636

## Description

### Technical Field

The invention relates to a multi-function clamp, especially to a clamp for veterinarians that is compatible with many kinds of lead wires and applied to the equipment such as veterinary electrocardiograph, veterinary monitor and so on.

### Background Art

Clamp for veterinarians currently mainly used in electrocardiograph, veterinary monitors and other products, which clipped in the animal's skin to detect the animal life signs. A Chinese patent issued number CN201312804Y discloses a clamp includes a upper clip 10, a lower clip 90, a clamping shaft 102 connected between the upper clip 10 and the lower clip 90, and a connecting post 100 located on the upper clip 10 and configured to secure a lead wire, as shown in figure 1 and figure 2 of the patent CN201312804Y. Another Chinese patent issued number CN85202339U also discloses a clamp includes a upper clip 12, a lower clip 13, a clamping shaft 11 connected between the upper clip 12 and the lower clip 13, and an electrode part 19 clamping two sides of the upper clip 12 and configured to secure a lead wire, as shown in figure 3 of the patent CN85202339U. Apparently, the structures of the clamps of the patents CN201312804Y and CN85202339U are capable of securing only one type lead wire. If a pet requires to use different types of lead wires, such as probe-type lead wires, banana plug lead wires,snap-type lead wires,and clip-type lead wires, the satisfaction could not be fulfilled.

### Contents of the Invention

Aiming at the shortcomings above in existing technology, the invention is to provide a multi-function clamp for veterinarians which is compatible with many kinds of lead wires, decreases the procurement cost for the accessories, increasing the efficiency in usage of the clamp for veterinarians and greatly facilitates the operation.

A multi-function clamp for veterinarians includes a clamp body including an upper clip, a lower clip, a bolt and a torsional spring. The clamp body is installed with a connection assembly connectable to lead wires. The connection assembly includes several installation sections which can match with different types of lead wire plugs.

The connection assembly includes a dual-bore copper and a chamfer head screw. The chamfer head screw passes through the lower clip to install with the dual-bore copper, so as to install the dual-bore copper in the lower clip.

The bottom of the dual-bore copper includes a snap, wherein both sides of the dual-bore copper defines a pair of mounting holes having a diameter of 3mm and 4mm respectively. One of the pair of mounting holes is perpendicular to another one of the pair of mounting holes in the axial direction of the holes.

The connection assembly includes a chamfer head screw, a single-hole copper and a hand screw, wherein the chamfer head screw passes through the lower clip to install with the single-hole copper, so as to lock the single-hole copper in the lower clip.

The side face of the single-hole copper defines a mounting hole having a diameter of 4mm, wherein the mounting hole installs with the lead wire plug and the hand screw is screwed into the single-hole copper, while the hand screw tightly fixes the lead wire plug on the single-hole copper.

The bottom of the hand screw is a snap.

The connection assembly includes a double-headed electrode socket and a rivet wherein the rivet matches with the double-headed electrode socket, so as to install the double-headed electrode socket in the lower clip.

The double-headed electrode socket defines a pair of mounting holes having a diameter of 3mm and 4mm respectively, and located at two flanks of the double-headed electrode socket respectively, while the bottom of the rivet is a snap.

The lower clip includes a plurality of first teeth, and the connection assembly is located at a tail end of the lower clip.

A tail end of the upper clip includes a plurality of wavy stripes, and a front end of the upper clip defines multiple dots, and the upper clip includes a plurality of second teeth engaging with the first teeth on the lower clip.

By adopting the compatibility design, the multi-function clamp for veterinarian could apply to wider areas, a pet clinic equipped with the multi-function clamp for veterinarians is applicable even using the probe-type lead wires, the banana plug lead wires, the lead wires with snap and the clip-type lead wires simultaneously, which greatly decreases the procurement cost for equipment and the accessories, and easier to be operated. Furthermore, by adopting the anti-slip design, the multi-function clamp for veterinarian will improve remarkably for the matter the clamp drops off from the operators or the animal skins, which saves time in operation.

### Description of Figures

Fig.1 is the whole structure drawing of embodiment 1 of the invention;
Fig.2 is the assembly diagram of the connection assembly and the clamp of Fig.1;
Fig.3 is the exploded view of the embodiment 1;
Fig.4 is the whole structure drawing of embodiment 2 of the invention;
Fig.5 is the assembly diagram of the connection assembly and the clamp of Fig.4;
Fig.6 is the exploded view of the embodiment 2;
Fig.7 is the whole structure drawing of embodiment 3 of the invention;
Fig.8 is the assembly diagram of the connection assembly and the clamp of Fig.7;
Fig.9 is the exploded view of the embodiment 3.

### Mode of Carrying out the Invention

Further explanation to the invention is stated below by three embodiment of implementation combining with the attached figures:

### Embodiment 1:

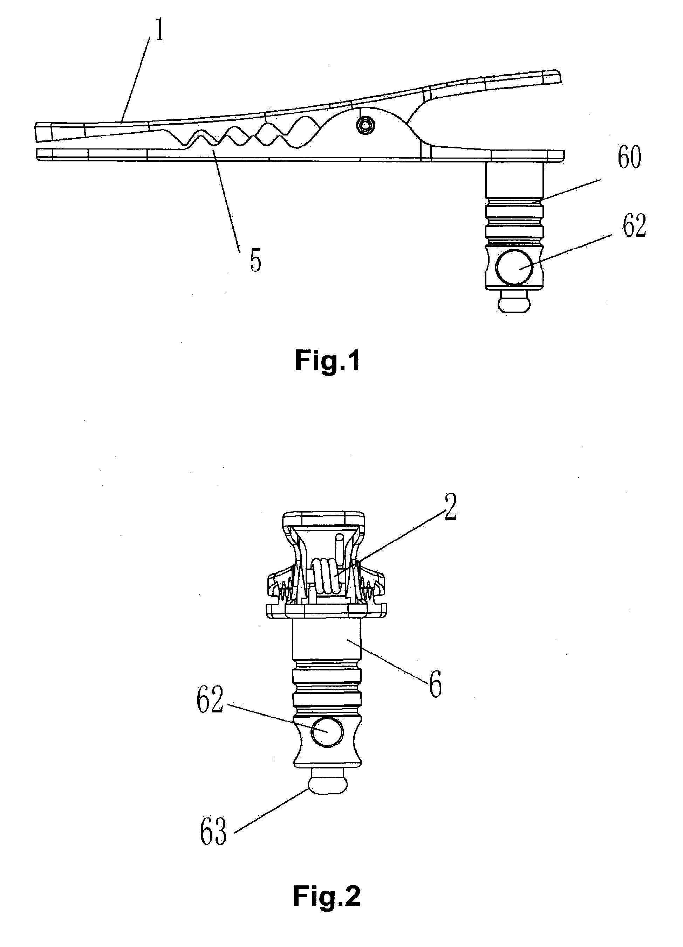

The multi-function clamp for veterinarians as shown in Fig.1, Fig.2 and Fig.3 is comprised by upper clip 1, lower clip 5, bolt 3, torsional spring 2 and the connection assembly, where, the connection assembly includes a dual-bore copper 6 wherein the dual-bore copper 6 has two mounting holes and one M3*6 chamfer head screw 4; when assembling, put the lower clip 5 in a plane surface, install the M3*6 chamfer head screw 4 with the dual-bore copper 6 by passing through the lower clip 5, then lock the dual-bore copper 6 in the lower clip 5, at the time the torsional spring 2 is placed in the lower clip 5, cover the upper clip 1 and put on the bolt 3, then press-riveting the upper clip 1 and the lower clip 5 to form an integral clamp for veterinarian. Thereinto, the tail end of the upper clip 1 has wavy stripes 12, for which the main purpose of the stripes is to prevent the clamp drop from the users; the front end of the upper clip 1 has multiple circular dots 11, and between the upper clip 1 and the lower clip 5 has a plurality of second teeth 13 and a plurality of first teeth 51 engaging with the second teeth 13 in separately to prevent the clamp loosing from the animal skins.

The embodiment applies to different types of lead wires, the dual-bore copper 6 has mounting holes 61 with a diameter of 3mm and mounting holes62 with a diameter of 4mm, and at the bottom of the dual-bore copper 6 has snap plug 63 with a diameter of 4mm; when the user using the electrocardiogram (hereinafter refers as "ECG") cable, if it is a probe-type with a diameter of 3mm, insert the lead wires into the mounting holes 61 with a diameter of 3mm for normal usage; if the ECG cable is banana plug type with a diameter of 4mm, insert the lead wires into the mounting holes 62 with a diameter of 4mm for normal usage; if the ECG cable is snap-type with a diameter of 4mm, insert the lead wires into the snap plug 63 with a diameter of 4mm for normal usage.

### Embodiment 2:

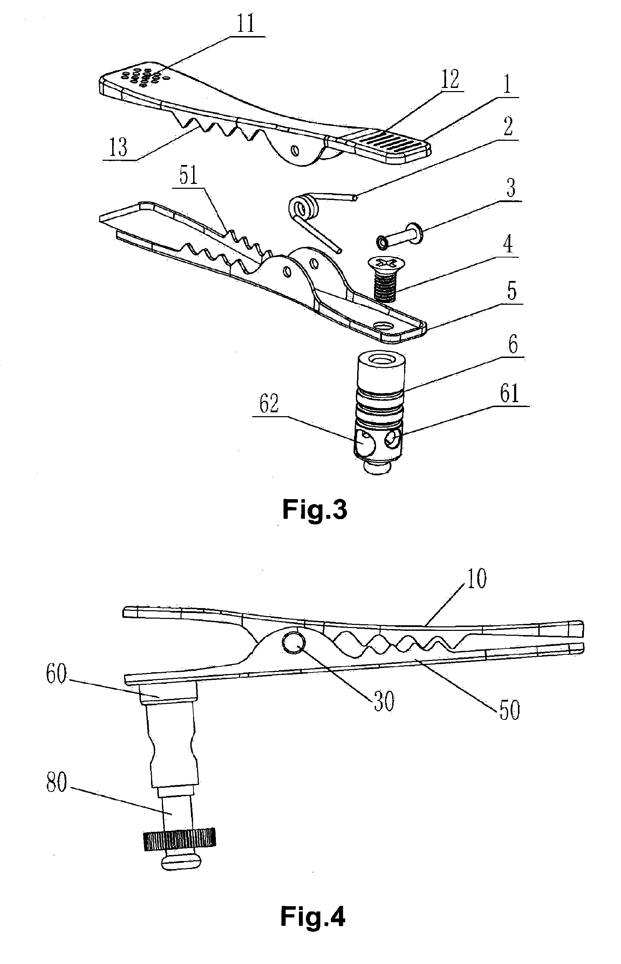

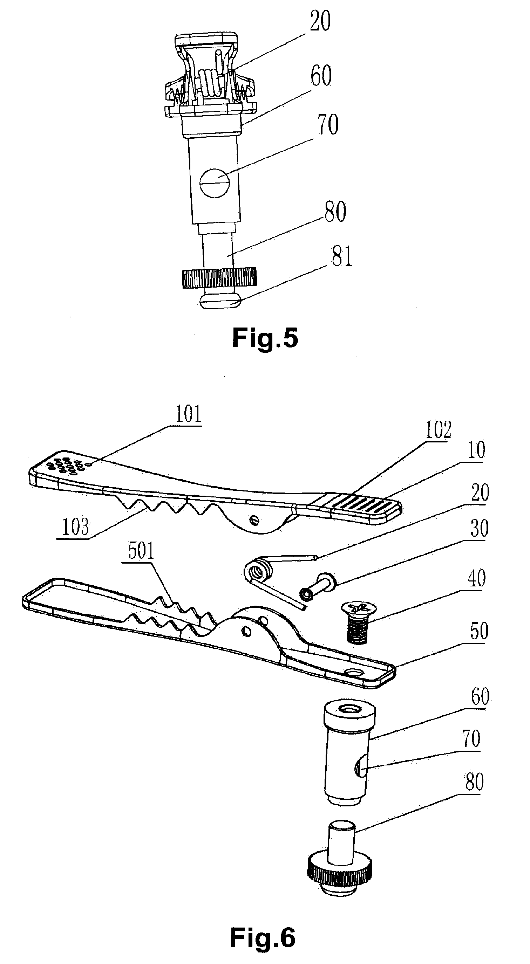

The multi-function clamp for veterinarians as shown in Fig.4, Fig.5 and Fig.6 is comprised by upper clip 10, lower clip 50, bolt 30, torsional spring 20 and the connection assembly, where, the connection assembly includes a M3*6 chamfer head screw 40, single-hole copper 60 and hand screw 80; when assembling, put the lower clip 50 in a plane surface, install the M3*6 chamfer head screw 40 with the single-hole copper 60 by passing through the lower clip 50, then lock the single-hole copper 60 in the lower clip 50, at the time the torsional spring 20 is placed inside the lower clip 50, cover the upper clip 10 and put on the bolt 30, then press-riveting the upper clip 10 and the lower clip 50, at last screw the hand screw 80 into the single-hole copper 60. The single-hole copper 60 of the embodiment has mounting holes 70 with a diameter of 4mm and the hand screw 80 has snap plug 81 with a diameter of 4mm; when the user using the ECG cable, if it is a probe-type with a diameter of 3mm or banana plug type with a diameter of 4mm, insert the lead wires into the mounting holes 70 with a diameter of 4mm and then screw the hand screw 80 tightly to fix the lead wires for normal usage; if the ECG cable is snap-type with a diameter of 4mm, insert the lead wires into the snap plug 81 with a diameter of 4mm for normal usage.

Thereinto, the tail end of the upper clip 10 has wavy stripes 102, for which the main purpose of the stripes is to prevent the clamp drop from the users; the front end of the upper clip 10 has multiple circular dots 101, and between the upper clip 10 and the lower clip 50 has a plurality of second teeth 103 and a plurality of first teeth 501 engaging with the second teeth 103 in separately to prevent the clamp loosing from the animal skins.

### Embodiment 3:

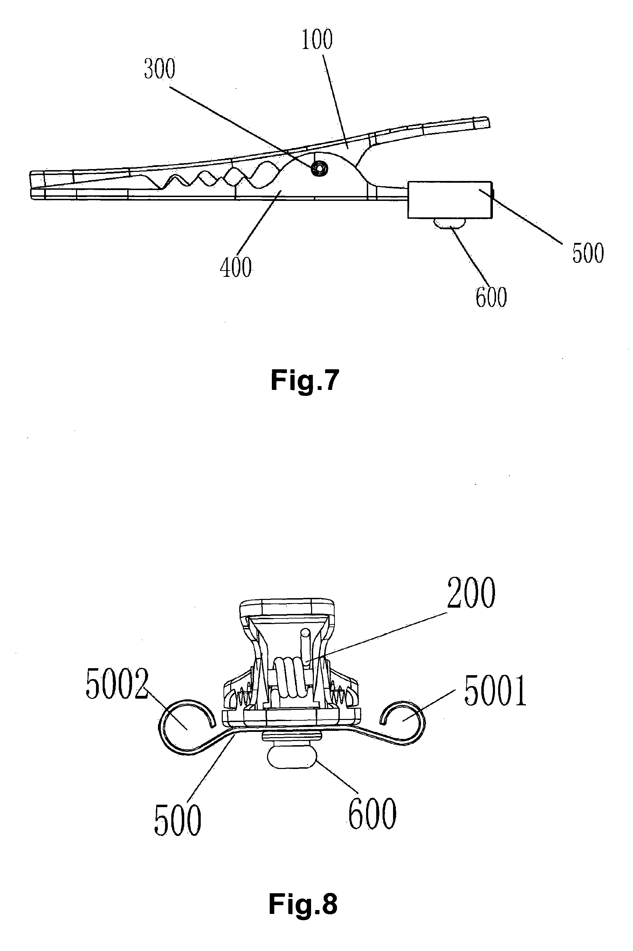

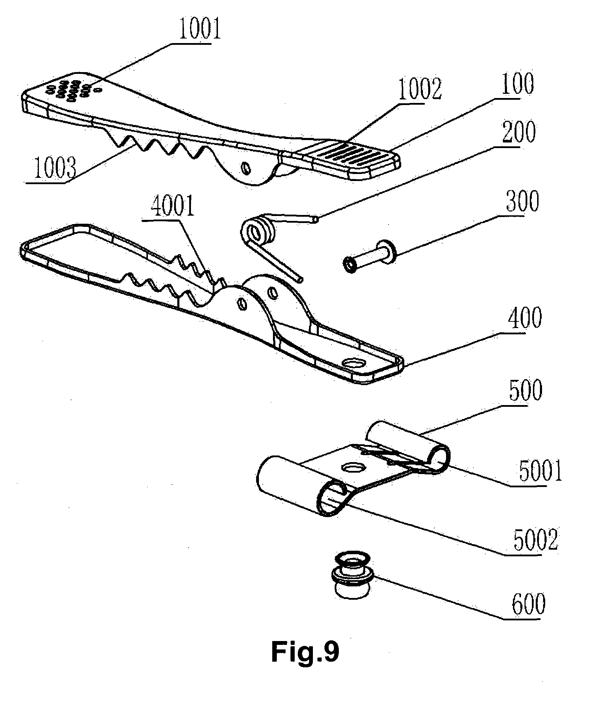

The multi-function clamp for veterinarians as shown in Fig.7, Fig.8 and Fig.9 is comprised by upper clip 100, lower clip 400, bolt 300, torsional spring 200 and the connection assembly, where, the connection assembly includes a double-headed electrode socket 500 and rivet 600, wherein the bottom of the rivet 600 has a snap plug; when assembling the clamp for veterinarians, lay flat the lower clip 400, using the rivet 600 to press-rivet the double-headed electrode socket 500 in the lower clip 400, and put the torsional spring 200 in the lower clip 400, then cover the upper clip 100, pass through the bolt 300, and then press-rivet the upper clip 100 and the lower clip 400. Thereinto, the tail end of the upper clip 100 has wavy stripes 1002, for which the main purpose of the stripes is to prevent the clamp drop from the users; the front end of the upper clip 100 has multiple circular dots 1001, and between the upper clip 100 and the lower clip 400 has a plurality of second teeth 1003 and a plurality of first teeth 4001engaging with the second teeth 1003 in separately to prevent the clamp loosing from the animal skins. The double-headed electrode socket 500 of the embodiment has probe holes 5001 with a diameter of 3mm and probe holes 5002 with a diameter of 4mm, and at the bottom of the snap-rivet 600 has snap plug 63 with a diameter of 4mm; when the user using the ECG cable, if it is a probe-type with a diameter of 3mm, insert the lead wires into the probe holes 5001 for normal usage; if the ECG cable is banana plug type with a diameter of 4mm, insert the lead wires into the probe holes 5002 with a diameter of 4mm for normal usage; if the ECG cable is snap-type with a diameter of 4mm, insert the lead wires into the snap plug with a diameter of 4mm for normal usage.

## Claims

1. A multi-function clamp for veterinarians, comprising a clamp body comprising an upper clip (1, 10, 100), a lower clip (5, 50, 400), a bolt (3, 30, 300) and a torsional spring (2, 20, 200); the clamp body being installed with a connection assembly connectable to lead wires, **characterized in that** the connection assembly comprises several installation sections which can match with different types of lead wire plugs.

2. The multi-function clamp for veterinarians according to claim 1, **characterized in that** the connection assembly comprises a dual-bore copper (6) and a chamfer head screw (4, 40), the chamfer head screw (4, 40) passes through the lower clip (5, 50, 400) to install with the dual-bore copper (6), so as to install the dual-bore copper (6) in the lower clip (5, 50, 400).

3. The multi-function clamp for veterinarians according to claim 2, **characterized in that** the bottom of the dual-bore copper (6) comprises a snap, wherein side of the dual-bore copper (6) defines a pair of mounting holes (61, 62) having a diameter of 3mm and 4mm respectively, while the one of the pair of mounting holes (61, 62) is perpendicular to another one of the pair of mounting holes (61, 62) in the axial direction of the holes.

4. The multi-function clamp for veterinarians according to claim 1, **characterized in that** the connection assembly comprises a chamfer head screw (4, 40), a single-hole copper (60) and a hand screw (80), wherein the chamfer head screw (4, 40) passes through the lower clip (5, 50, 400) to install with the single-hole copper (60), so as to lock the single-hole copper (60) in the lower clip (5, 50, 400).

5. The multi-function clamp for veterinarians according to claim 4, **characterized in that** the side face of the single-hole copper (60) defines a mounting hole (70) having a diameter of 4mm, wherein the mounting hole (70) installs with the lead wire plug and the hand screw (80) is screwed into the single-hole copper (60), while the hand screw (80) tightly fixes the lead wire plug on the single-hole copper (60).

6. The multi-function clamp for veterinarians according to claim 4 or claim 5, **characterized in that** the bottom of the hand screw (80) is a snap.

7. The multi-function clamp for veterinarians according to claim 1, **characterized in that** the connection assembly comprises a double-headed electrode socket (500) and a rivet (600), wherein the rivet (600) matches with the double-headed electrode socket (500), so as to install the double-headed electrode socket (500) in the lower clip (5, 50, 400).

8. The multi-function clamp for veterinarians according to claim 7, **characterized in that** the double-headed electrode socket (500) defines a pair of mounting holes (5001, 5002) having a diameter of 3mm and 4mm respectively, and located at two flanks of the double-headed electrode socket (500) respectively, while the bottom of the rivet (600) is a snap.

9. The multi-function clamp for veterinarians according to claim 1, **characterized in that** the lower clip (5, 50, 400) comprises a plurality of first teeth (51, 501, 4001), and the connection assembly is located at a tail end of the lower clip (5, 50, 400).

10. The multi-function clamp for veterinarians according to claim 9, **characterized in that** a tail end of the upper clip (1, 10, 100) comprises a plurality of wavy stripes (12, 102, 1002), and a front end of the upper clip (1, 10, 100) defines multiple dots (11, 101, 1001), and the upper clip (1, 10, 100) comprises a plurality of second teeth (13, 103, 1003) engaging with the first teeth (51, 501, 1003) on the lower clip (5, 50, 400).

## Patentansprüche

1. Mehrzweckklammer für Tierärzte mit einen Klemmkörper, der eine obere Klammer (1, 10, 100), eine untere Klammer (5, 50, 400), einen Bolzen (3, 30, 300) und eine Torsionsfeder (2, 20, 200) umfasst; wobei der Klemmkörper mit einer Verbindungsanordnung anschließbar Anschlussdrähte installiert, **dadurch gekennzeichnet, dass** die Verbindungsanordnung mehrere Installationsabschnitt umfasst, die mit verschiedenen Arten von Kabelsteckern übereinstimmen.

2. Die Mehrzweckklammer für Tierärzte nach Anspruch 1, **dadurch gekennzeichnet, dass** die Verbindungsanordnung eine Dual-Bohrung Kupfer (6) und eine Senkkopfschraube (4, 40), die Senkkopfschraube (4, 40) durchläuft durch die untere Klammer (5, 50, 400) dann in Eingriff mit dem Dual-Bohrung Kupfer (6), um so die Dual-Bohrung Kupfer (6) in der unteren Klammer (5, 50, 400) zu installieren.

3. Die Mehrzweckklammer für Tierärzte nach Anspruch 2, **dadurch gekennzeichnet, dass** der Boden des Dual-Bohrung Kupfer (6) ein schnapp-befestigungseinrichtung umfasst, wobei Seite der Dual-Bohrung Kupfer (6) ein Paar von Befestigungslöchern (61, 62) mit einem Durchmesser von 3 mm und 4 mm umfasst, während ein Paar von Befestigungslöchern (61, 62) senkrecht zu einem anderen Paar von Befestigungslöchern (61, 62) in axialer Richtung der Bohrungen angeordnet ist.

4. Die Mehrzweckklammer für Tierärzte nach Anspruch 1, **dadurch gekennzeichnet, dass** die Verbindungsanordnung eine Senkkopfschraube (4, 40), eine Kupferteil (60) an einem einzigen Loch und eine Schraube (80) zur manuellen Betätigung aufweist, wobei die Senkkopfschraube (4, 40) durchläuft durch den unteren Element (5, 50, 400), dann in Eingriff kommt mit dem Kupferteil (60) mit einem einzigen Loch, um so die Kupferteil (60) mit einem einzigen Loch in der unteren Klammer (5, 50, 400) verriegelt ist.

5. Die Mehrzweckklammer für Tierärzte nach Anspruch 4, **dadurch gekennzeichnet, dass** die Seitenfläche der Kupferteil (60) mit einem einzigen Loch definiert eine Montageöffnung (70) mit einem Durchmesser von 4 mm, wobei das Montageloch (70) installiert mit dem Leitungsdraht Stecker und die Schraube (80) zur manuellen Betätigung in die Kupferteil (60) mit einem einzigen Loch eingeschraubt ist, während die Schraube (80) zur manuellen Betätigung den Bleidraht Stecker auf die Kupferteil (60) mit einem einzigen Loch fixiert ist.

6. Die Mehrzweckklammer für Tierärzte nach Anspruch 4 oder Anspruch 5, **dadurch gekennzeichnet, dass** die Schraube (80) zur manuellen Betätigung umfasst ein schnapp-befestigungseinrichtung.

7. Die Mehrzweckklammer für Tierärzte nach Anspruch 1, **dadurch gekennzeichnet, dass** die Verbindungsanordnung eine Doppelkopf Elektrodenbuchse (500) und ein Niet (600), wobei die Niete (600) mit der Doppelkopf Elektrodenbuchse (500) übereinstimmt, um so die Montage von der Doppelkopf Elektrodenbuchse (500) in der unteren Klammer (5, 50, 400) realisiert ist.

8. Die Mehrzweckklammer für Tierärzte nach Anspruch 7, **dadurch gekennzeichnet, dass** der Doppelkopf Elektrodenbuchse (500) definiert ein Paar von Befestigungslöchern (5001, 5002) mit einem Durchmesser von 3 mm und 4 mm und auf zwei sich Flanken der Doppelkopf Elektrodenbuchse (500), während der Boden der Niete (600) ist ein schnapp-befestigungseinrichtung.

9. Die Mehrzweckklammer für Tierärzte nach Anspruch 1, **dadurch gekennzeichnet, dass** die untere Klammer (5, 50, 400) umfasst eine Mehrzahl von ersten Zähnen (51, 501, 4001), und die Verbindungsanordnung an einem Heck Ende der unteren Klammer (5, 50, 400) befindet ist.

10. Die Mehrzweckklammer für Tierärzte nach Anspruch 9, **dadurch gekennzeichnet, dass** ein hinteres Ende der oberen Klemme (1, 10, 100) umfasst eine Mehrzahl von gewellten Streifen (12, 102, 1002), und dass ein vorderes Ende die obere Klammer (1, 10, 100) eine Vielzahl Punkte (11, 101, 1001) aufweist, die obere Klammer (1, 10, 100) umfasst eine Vielzahl von zweiten Zähnen (13, 103, 1003) und kommt in Eingriff mit den ersten Zähnen (51, 501, 1003) an der unteren Klemme (5, 50, 400).

## Revendications

1. Une pince multifonctionnelle pour vétérinaires, comprenant un corps de pince comprenant un élément supérieur (1, 10, 100), un élément inférieur (5, 50, 400), un boulon (3, 30, 300) et un ressort de torsion (2, 20, 200) ; le corps de pince comportant un ensemble de connexion pouvant être connecté à des fils conducteurs, **caractérisée en ce que** l'ensemble de connexion comporte plusieurs portions d'installation qui sont adaptables à différents types de connecteurs de câble.

2. La pince multifonctionnelle pour vétérinaires selon la revendication 1, **caractérisée en ce que** l'ensemble de connexion comprend une pièce en cuivre (6) à double alésage et une vis (4, 40) à tête fraisée, la vis (4, 40) à tête fraisée passant à travers l'élément inférieur (5, 50, 400) puis venant en engagement avec la pièce en cuivre (6) à double alésage, de manière à monter la pièce en cuivre (6) à double alésage sur l'élément inférieur (5, 50, 400).

3. La pince multifonctionnelle pour vétérinaires selon la revendication 2, **caractérisée en ce que** la partie inférieure de la pièce en cuivre (6) à double alésage comprend une portion à encliquetage, un côté de la pièce en cuivre (6) à double alésage définissant une paire de trous de montage (61, 62) ayant respectivement un diamètre de 3 mm et 4 mm, l'un des trous de montage (61, 62) de la paire de trous étant perpendiculaire à l'autre trous de montage (61, 62) de la paire de trous selon la direction axiale des trous.

4. La pince multifonctionnelle pour vétérinaires selon la revendication 1, **caractérisée en ce que** l'ensemble de connexion comprend une vis à tête fraisée (4, 40), un pièce en cuivre (60) à un seul trou et une vis (80) à actionnement manuel, la vis à tête fraisée (4, 40) passant à travers l'élément inférieur (5, 50, 400) puis venant en engagement avec la pièce en cuivre (60) à un seul trou, de manière à verrouiller la pièce en cuivre (60) à un seul trou dans l'élément inférieur (5, 50, 400).

5. La pince multifonctionnelle pour vétérinaires selon la revendication 4, **caractérisée en ce que** la face latérale de la pièce en cuivre (60) à un seul trou définit un trou de montage (70) ayant un diamètre de 4 mm, le trou de montage (70) étant apte à venir en engagement avec la fiche du câble conducteur et la vis (80) à actionnement manuel est vissée dans la pièce en cuivre (60) à un seul trou, la vis (80) à actionnement manuel fixant fermement la fiche du câble conducteur sur la pièce en cuivre (60) à un seul trou.

6. La pince multifonctionnelle pour vétérinaires selon la revendication 4 ou la revendication 5, **caractérisée en ce que** la portion inférieure de la vis (80) à actionnement manuel est à encliquetage.

7. La pince multifonctionnelle pour vétérinaires selon la revendication 1, **caractérisée en ce que** l'ensemble de connexion comprend une douille formant électrode à double tête (500) et un rivet (600), le rivet (600) correspondant à la douille formant électrode à double tête (500) de manière à réaliser le montage de la douille formant électrode à double tête (500) sur l'élément inférieur (5, 50, 400).

8. La pince multifonctionnelle pour vétérinaires selon la revendication 7, **caractérisée en ce que** la douille formant électrode à double tête (500) définit une paire de trous de montage (5001, 5002) ayant respectivement un diamètre de 3 mm et de 4 mm et situés l'un et l'autre sur les deux flancs de la cavité d'électrode à double tête (500), la portion inférieure du rivet (600) étant à encliquetage.

9. La pince multifonctionnelle pour vétérinaires selon la revendication 1, **caractérisée en ce que** l'élément inférieur (5, 50, 400) comprend une pluralité de premières dents (51, 501, 4001), et **en ce que** l'ensemble de connexion est situé à une extrémité postérieure de l'élément inférieur (5, 50, 400).

10. La pince multifonctionnelle pour vétérinaires selon la revendication 9, **caractérisée en ce qu'**une extrémité postérieure de l'élément supérieur (1, 10, 100) comprend une pluralité de bandes ondulées (12, 102, 1002), et **en ce qu'**une extrémité antérieure de l'élément supérieur (1, 10, 100) définit de multiples points (11, 101, 1001), l'élément supérieur (1, 10, 100) comprenant une pluralité de deuxièmes dents (13, 103, 1003) venant en prise avec les premières dents (51, 501, 1003) sur l'élément inférieur (5, 50, 400).
